# EUROPEAN PATENT SPECIFICATION

(11) **EP 3 146 894 B1**
(45) Date of publication and mention of the grant of the patent: **13.05.2026**
(21) Application number: 15306502.4
(22) Date of filing: 28.09.2015
(51) Int. Cl.: A61B 5/00, A61B 5/11

(54) **A USER MIGRAINE ANALYSIS COMPONENT**
BENUTZERMIGRÄNEANALYSEKOMPONENTE
COMPOSANT D'ANALYSE DE MIGRAINE D'UTILISATEUR

(43) Date of publication of application: 29.03.2017
(73) Proprietor: Essilor International, 94220 Charenton-le-Pont (FR)
(72) Inventor: BARRAU, Coralie, 94220 CHARENTON-LE-PONT (FR); ROUSSEAU, Denis, 94220 CHARENTON-LE-PONT (FR); LORE, Marie, 94220 CHARENTON-LE-PONT (FR); VILLETTE, Thierry, 94220 CHARENTON-LE-PONT (FR)
(74) Representative: Cabinet Novitech

(56) References cited:
- WO-A1-2015/127441
- WO-A2-2006/017153
- WO-A2-2006/086086
- US-A1- 2001 028 309
- US-A1- 2005 154 419

## Description

### FIELD OF THE INVENTION

The invention relates to a user migraine analysis component and to a real-time visual behavior measuring device. The invention further relates to a system for determining the migraine risk for a user.

### BACKGROUND OF THE INVENTION

Migraines affect around 15% of the worldwide population. Half of migraine sufferers do not consult a physician when they feel impaired.

Thus, determining the risk of migraines for a user may be useful in many situations.

For example, some activities require a great degree of concentration. It is useful to be able to provide an indication of the migraine risk to the user carrying out such activity, or to a third party. Indeed, it has been observed that a change in behavior upon carrying out an activity may lead to very different results in terms of achievement of the activity.

Typically, when a person is driving it can be very useful to analyze the risk and the severity of migraine of the person so as to provide an alert when the risk or severity for a migraine is greater than a threshold value and driving represents a risk.

Therefore, there is a need for a device and a method for accurately determining the risk or the severity of migraine of a person.

One object of the present invention is to provide such a device.

WO2006/017153 discloses an apparatus for determining migraine triggers.

### SUMMARY OF THE INVENTION

The invention is defined in the appended claims. It is disclosed a user migraine analysis component, comprising:
- a memory configured to store computer executable instructions; and
- a processor for executing the computer executable instructions, wherein the computer executable instructions comprise instructions for:
   - receiving at least one visual behavior parameter indicative of the real time visual behavior of the user, and
   - determining the migraine risk of the user based on the analysis of the at least one visual behavior parameter.

In particular, the memory may also be configured to store parameters.

Advantageously, determining the migraine risk of the user based on the analysis of at least one visual behavior parameter of the user provides a highly accurate estimate of the risk or the severity of migraine of the user.

Indeed, the inventors have observed that the real time visual behavior of a person may be linked very precisely and accurately to the migraine risk of the user.

According to embodiments which can be considered alone or in any combination:
- the visual behavior parameter relates at least to the eyelids activity of the user; and/or
- the computer executable instructions further comprise instructions for:
   - receiving real-time physiological data relating to at least one real time parameter of the physiology of the user, and
   - determining the migraine risk considering the real-time physiological data; and/or
- the real-time parameter of the physiology of the user relates to sweating of the user and/or the pulse of the user and/or the temperature of the user and/or the breathing rhythm of the user and/or muscle spasm of the user; and/or
- the computer executable instructions further comprise instructions for:
   - receiving real-time environment data relating to at least one real-time parameter of the environment of the user, and
   - determining the migraine risk considering the real-time environment data; and/or
- the real-time parameter of the environment of the user relates to the features of the light received by the user, said features comprising at least one of the temporal features, the spatial features, the spectral features, and the intensity of the light; and/or
- the real-time parameter of the environment of the user relates to temperature and/or the noise of the environment of the user and/or the time of the day; and/or
- the computer executable instructions further comprise instructions for:
   - receiving life habits data and/or health profile data relating to at least one parameter of the life habits of the user and/or to at least one parameter of the health profile of the user, and
   - adjusting the migraine risk based on the life habits data and/or on the health profile data; and/or
- the parameter of the life habits of the user relates to the food habits of the user and/or the physical activity habits of the user and/or the rhythm of life of the user, and the parameter of the health profile of the user relates to the professional situation of the user and/or the personal situation of the user and/or the general state of health of the user and/or the gender of the user and/or the age of the user and/or the ongoing migraine treatment of the user; and/or
- the computer executable instructions further comprise instructions for:
   ∘ providing at least one migraine history parameter indicative of the migraine history of the user, and
   ∘ adjusting the migraine risk based on at least one migraine history parameter; and/or
- the parameter of migraine history is determined with measurements and/or with answers of the user to questionnaires.

The invention relates to a real-time visual behavior measuring device comprising at least one sensor configured to measure in real time at least one visual behavior parameter indicative of the visual behavior of the user and a communication unit configured to communicate the measured real time visual behavior parameter to a user migraine analysis component according to the invention.

The real-time visual behavior measuring device may be a head mounted device arranged to be mounted on the head of the user.

The invention relates to a system for determining the migraine risk of a user, the system comprising:
- a real-time visual behavior measuring device according to the invention, and
- a user migraine risk analysis component according to the invention.

The present disclosure further relates to a method, not being part of the invention, for determining the migraine risk for a user, the method comprising:
- a real-time visual behavior measuring step, during which at least one visual behavior parameter indicative of the visual behavior of the user is measured in real time, and
- an analyzing step, during which the migraine risk is determined based on the analysis of the at least one visual behavior parameter.

The invention further relates to a computer program product comprising one or more stored sequences of instructions that are accessible to a processor and which, when executed by the processor, causes the processor to carry out the steps of the method disclosed.

The invention also relates to a computer-readable storage medium having a program recorded thereon; where the program makes the computer execute the method disclosed.

The invention further relates to a device comprising a processor adapted to store one or more sequence of instructions and to carry out at least one of the steps of the method disclosed.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or a Digital Signal Processor ("DSP") which can act as a general processor or a FPGA selectively activated or reconfigured by a computer program stored in the computer which can act as a co-processor when configured by a local processor or memory, or like a custom processor working alone and configured by local memory. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), ferroelectric random access memories (FRAM), magnetic or optical cards, or any other type of media suitable for storing electronic instructions, and capable of being coupled to a computer system bus. The apparatus may also be a microcontroller, for example a chip comprising a central processing unit (CPU), a memory, sensors, and an interface for external sensors. All functions may be comprised in the same silicon chip, or in the same package with the system in package (SIP) technology, where several chips may be connected together inside the package.

The processes and displays presented herein are not inherently related to any particular computer or other apparatus. Various general purpose systems may be used with programs in accordance with the teachings herein, or it may prove convenient to construct a more specialized apparatus to perform the desired method.

The desired structure for a variety of these systems will appear from the description below. In addition, embodiments of the present invention are not described with reference to any particular programming language. It will be appreciated that a variety of programming languages may be used to implement the teachings of the inventions as described herein.

### BRIEF DESCRIPTION OF THE DRAWINGS

Embodiments of the invention will now be described, by way of example only, and with reference to the following drawings in which:
- Figure 1 is a schematic representation of a user migraine analysis component according to the invention;
- Figure 2 is an illustration of a chart-flow of a method carried out by the processor according to the
   invention;
- Figure 3 is a representation of a real-time behavior measuring device according to the invention; and
- Figure 4 represents a global database synoptic for a user migraine analysis component according to the invention.

### DETAILED DESCRIPTION OF PREFERRED EMBODIMENTS

Elements in the figures are illustrated for simplicity and clarity and have not necessarily been drawn to scale. For example, the dimensions of some of the elements in the figure may be exaggerated relative to other elements to help improve the understanding of the embodiments of the present invention.

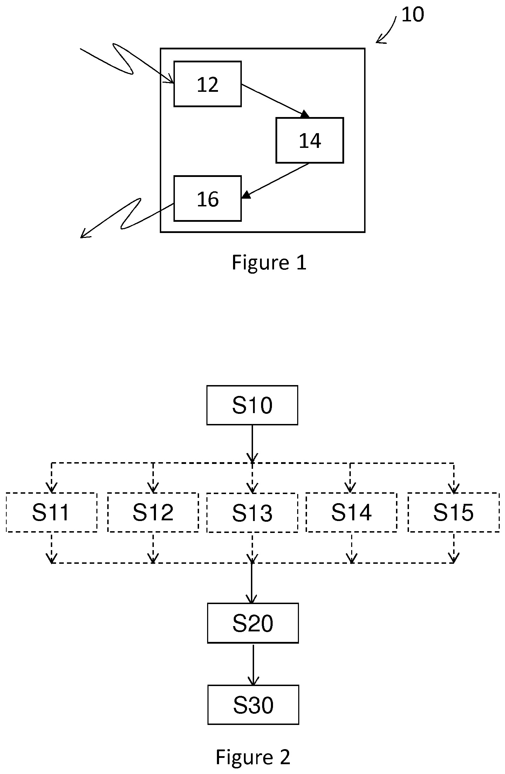

As illustrated in figure 1, the invention relates to a user migraine analysis component 10. The migraine analysis component comprises:
- a communication module 12,
- a memory 14, and
- a processor 16.

The communication module 1 is arranged to receive The data are real-time visual behavior data indicative of a visual behavior parameter of the user of the migraine analysis component.

The real-time visual behavior data are typically measured by sensors and sent to the communication module 12. The sensors may be connected by wires to the communication module, or the communication may be wireless. The wireless communication can use different communication protocols such as Bluetooth, Zigbee, WiFi or others.

The communication module may further receive life habits data relating to at least one parameter of the life habits of the user of the migraine analysis component.

The communication module may further receive health profile data relating to at least one parameter of the health profile of the user of the migraine analysis component.

The communication module may also receive sleep history data indicative of at least one sleep quality parameter.

The life habits data and/or the sleep history data may be stored in a distant entity. The distant entity can include different storing objects such as personal digital assistants, audio/video devices, mobile phones, MPEG-1 Audio Layer 3 (MP3) players, personal computers, laptops, tablets, Bluetooth headset, watch, wristband, etc...

The communication with the distant entity is usually wireless communication or via the Internet, for example with a WiFi interface. The distant entity can also include Web servers, file servers, media servers, etc. with which the communication module communicates via any of a number of known protocols, such as the hypertext transfer protocol (HTTP).

The memory 14 stores computer executable instructions that are to be executed by the processor 16.

The memory 14 may store life habits data relating to at least one parameter of the life habits of the user of the migraine analysis component.

The memory 14 may also store sleep history data indicative of at least one sleep quality parameter of the user of the migraine analysis component. The processor 16 is configured to execute at least part of the computer executable instructions stored in the memory 14.

The computer executable instructions comprise instructions for having the processor carry out a method disclosed.

As illustrated on figure 2, a method comprises at least:
- a real-time visual behavior parameter receiving step S10, and
- an analyzing step S20.

During the real-time visual behavior parameter receiving step S10, at least one visual behavior parameter indicative of the visual behavior of the user is received in real time. Typically, the visual behavior parameter is measured in real time by at least one sensor and the measured parameter is sent in real time to the user migraine analysis component.

For example, the visual behavior parameter may relate at least to the eyelids activity of the user, such as opening frequency and force or shift in the near point of convergence. As another example, the visual behavior parameter may relate at least to the eyebrows activity of the user, such as frequency and force of frown.

In the sense of the invention, "real-time" refers to the fact of measuring and sending the visual behavior parameter of the user at the same time or with a shift smaller than or equal to a few seconds.

During the analyzing step S20, the migraine risk is determined based on the analysis of the at least one visual behavior parameter.

As illustrated on figure 2, the method may further comprise:
- a real-time physiological data measuring step S11, and/or
- a real-time environment data measuring step S12, and/or
- a life habits data and/or health profile data providing step S13.

During the real-time physiological data measuring step S11, real-time physiological data relating to at least one parameter of the physiology of the user are measured in real time. During the analyzing step S20, the migraine risk may be determined considering the real-time physiological data.

The real-time parameter of the physiology of the user may relate to sweating of the user and/or to the pulse of the user and/or to the temperature of the user and/or to the breathing rhythm of the user and/or to muscle spasm of the user.

The real-time environment of the user may also be considered when determining the migraine risk. During the real-time environment data measuring step S12, real-time environment data relating to at least one parameter of the environment of the user are measured in real time. During the analyzing step S20, the migraine risk may be determined considering the real-time environment data.

For example, the parameter of the environment of the user may relate to the features of the light received in real time by the user, said features comprising at least one of the temporal features, the spatial features, the spectral features, and the intensity of the light. In particular, in a bright environment, the migraine risk of the user may be increased.

Further parameters of the environment of the user may be considered as the temperature and/or the noise of the environment of the user and/or the time of the day.

Life habits of the user may be of interest when determining/predicting the migraine risk of the user. Moreover, the health profile of the user may affect the migraine risk of the user. During the life habits data and/or health profile data providing step S13, life habits data and/or health profile data respectively relating to at least one parameter of the life habits of the user and to at least one parameter of the health profile of the user are provided. During the analyzing step S20, the migraine risk is adjusted based on the life habits data and/or on the health profile data.

The health profile of the user typically relates to the professional situation of the user and/or the personal situation of the user and/or the general state of health of the user and/or the sex of the user and/or the age of the user and/or the ongoing migraine treatment of the user.

Typically, the life habits of the user relates to the food habits of the user and/or the physical activity habits of the user and/or the sleeping habits of the user and/or the rhythm of life of the user or any other life habits parameter that may have an influence on the migraine risk of the user.

The sleeping habits of the user may comprise sleep quality history of the user. For example, as illustrated on figure 2, the method may further comprise a sleep quality history parameter providing step S14, during which at least one sleep quality history parameter indicative of the sleep quality history of the user is provided to the user migraine analysis component. During the analyzing step S20, the migraine risk is adjusted based on the at least one sleep quality history parameter.

The sleep quality history parameter may be determined through subjective indication provided by the user. For example, the user may provide each morning an indication on the sleep quality of the night such as a grade depending on the user's feeling of his sleep quality.

The sleep quality history may be determined using objective measurements relating to the user when sleeping. For example, the inventors have observed that a sleep quality history parameter relating to the sleep cycles efficiency history of the user is highly relevant when determining the risk of migraine of the user.

The sleep quality history parameter may relate at least to sleeping physiological data relating to at least one parameter of the physiology of the user upon sleeping.

The sleeping physiological data may for example be obtained by measuring the breathing of the user upon sleeping and/or the movement of the user upon sleeping and/or to the pulse of the user upon sleeping and/or the sound produced by the user upon sleeping. EEG (Electroencephalography) and REM (Rapid Eye Movement) analysis can be used also to analyze sleep quality. Waking up time relative to REM and/or paradoxal sleep is an important parameter for sleep quality and health.

The real-time head movement of the user may also be considered when determining the severity of the migraine. During the real-time head movement data measuring step, real-time head movement data relating to the movement of the head of the user are measured and provided in real time. During the analyzing step S20, the migraine risk may be adjusted considering the real-time head movement data.

Typically, real-time head movement may be linked to the level of pain of a user. For example, head tilt may provide indication of an increase pain level.

The environment of the user while sleeping may influence the sleep quality of the user. Therefore, according to an embodiment of the invention, the sleep quality history parameter relates at least to sleeping environment data relating to at least one parameter of the environment of the user upon sleeping.

For example, the parameter of the environment of the user upon sleeping may relates to the temperature of the sleeping environment of the user and/or the noise of the sleeping environment of the user and/or the features of the light of the sleeping environment of the user received by the user, said the light of the sleeping environment comprise at least one of the temporal features, the spatial features, the spectral features, the intensity of the light.

To increase the accuracy of the migraine risk determination, the light of the sleeping environment may be combined with the timing in the sleep cycle upon which such light is received by the user.

The method may further comprise a screen time providing step during which data representative of the time a user spent watching a screen are provided. During the analyzing step S20, such screen time data may be considered to determine more accurately the migraine risk of the user. Some flash or alternative light patterns can provoke migraine or epilepsy. Light sensors on the glasses can detect these light patterns.

The migraine history may be considered to determine more accurately the migraine risk of the user and to possibly update it. As illustrated on figure 2, the method may further comprise a migraine history parameter providing step S15 during which at least one migraine history parameter indicative of the migraine history of the user is provided. The migraine history parameter may comprise data representative of the past level of pain of the user. During the analyzing step S20, the migraine risk is updated based on at least one migraine history parameter. In other words, the migraine risk may be adjusted based on at least one migraine history parameter.

In the invention, considering if the user has been having a high level of pain recently or not is to increase the accuracy of the method . So as to improve even more the accuracy and precision of the method, the method may comprise measuring and providing further real-time parameters.

The parameter of migraine history may be determined with measurements and/or with answers of the user to questionnaires. For example, the questionnaires may comprise questions about the frequency and the duration of the migraine. For example, the user may provide feedback concerning his current level of pain, for example using a scale of pain level.

The relevancy of each measured parameter may be adjusted based on the answers of the user to questionnaires, like with the MIDAS (Migraine Disability Assessment) score by scaling the severity of the migraine between grade I and grade IV.

Advantageously, such feedback makes it possible for a user to customize the analysis and therefore helps improve the accuracy of the method and/or the migraine analysis component.

The method may further comprise an information generating step S30 during which information based on said determined migraine risk of the user is generated.

The information may comprise a recommendation data based on the determined migraine risk of the user.

The information may further comprise an alert indicative of the determined migraine risk of the user.

The information may be induced according to a score value calculated taking into account each parameters and their interactions, and/or values history, and/or calibrated normal parameters such as the minimum, the maximum, or critical values.

The information may be generated, for example, in the user migraine analysis component or in the distant entity.

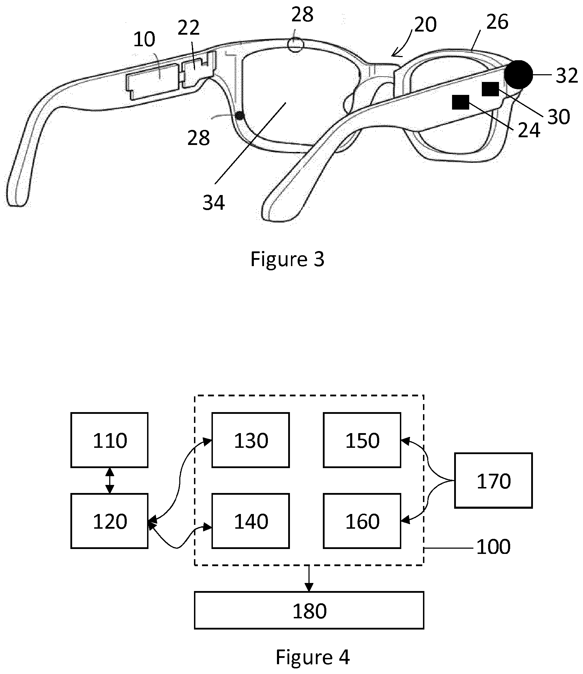

As represented on figure 3, the invention further relates to a real-time visual behavior measuring device 20. The real-time visual behavior measuring device comprises at least one sensor configured to measure in real time at least one visual behavior parameter indicative of the visual behavior of the user and a communication unit 22 configured to communicate the measured real time visual behavior parameter to a user migraine analysis component 10 according to the invention.

In the example represented in figure 3, the real-time visual behavior measuring device 20 is a head mounted device comprising a frame intended to be mounted on the head of the user.

Furthermore, in the example of figure 3, the user migraine analysis component 10 is embedded in the real-time visual behavior measuring device 20.

The real-time visual behavior measuring device 20 may comprise different type of sensors depending on the type of real-time parameters that are to be considered.

Typically, the real-time visual behavior measuring device 20 may comprise a head movement detection sensor 24 that may comprise an accelerometer and/or gyroscope and/or compass configured to sense the orientation and position and variation of orientation and position of the head of the user.

The head movement detection sensor 24 may be placed on the frame 26 of the real-time visual behavior measuring device 20, as illustrated on Figure 3.

The real-time visual behavior measuring device 20 may further comprise an eye behavior sensor, such as an eye tracking device 28 arranged to measure eye movements, pupil diameter and activity of the user. Eye behavior also includes eyelid movement detection, which can be done by infrared emitting LED and infrared sensor.

A heart beat sensor (not shown) and skin sensor (not shown) can also be included. A heart beat sensor can be an optical sensor or any other type of heart beat sensor. Skin sensor can be skin moisture and/or skin resistivity sensors, which can give accurate information about the wearer stress or pain level.

EEG and EOG (Electrooculography) activity can also be measured by skin sensors. These parameters are useful for sleep analysis, and can be worn only during sleeping time.

The real-time visual behavior measuring device 20 may further comprise a light sensor 30 arranged to measure features of light, such as temporal, spectral and intensity features.

The real-time visual behavior measuring device 20 may further comprise a mapping system 32, such as a GPS, arranged to provide features of the environment of the user.

The head mounted device 20 represented on figure 3 may also comprise two optical lenses 34. The optical lenses 34 may correspond to the optical prescription of the user. The optical lenses 34 may also be active lenses with an optical function which can be adapted for example to the user's needs. For example the lens may become darker if the light sensor detects a high luminosity and the monitoring system detects a risk of migraine due to high light condition.

The information based on the determined migraine risk of the user may be displayed on a display (not shown) of the head mounted device 20 or any other display in communication with the device that is configured to receive the information.

The communication unit 22 allows the local processor to receive information from different databases 100 illustrated on Figure 4. The communication unit 22 may allow the processor to receive information, for example stored in the user mobile phone 110 or in the cloud 120, containing parameters from external sensors 130 and/or health personal database 140, for example food habits, sleep quality or last risk exposure to migraine, that help the migraine analysis component to make the diagnostic of migraine risk and to provide an accurate decision and/or advice 180 for the user.

The communication unit 22 may also allow the processor to receive information containing experience or memorized parameters 150 and/or real time parameters 160 from embedded sensors 170, for example light, noise, temperature, eye movement, eyelid movement, activity, heartbeat of the user, blood pressure and oximetry of the user, time...

In another embodiment, the user migraine analysis component 10 may be in a distant entity communicating with the real-time visual behavior measuring device with the communication unit 22.

The distant entity can include different computing objects such as personal digital assistants, audio/video devices, mobile phones, MPEG-1 Audio Layer 3 (MP3) players, personal computers, laptops, tablets, Bluetooth headset, watch, wristband, etc.

The communication can be done through different communication devices and protocols, like Bluetooth, Zigbee, WiFi or others.

The invention has been described above with the aid of embodiments without limitation of the general inventive concept.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A user migraine analysis component (10), comprising:
- a memory (14) configured to store computer executable instructions; and
- a processor (16) for executing the computer executable instructions, wherein the computer executable instructions comprise instructions for:
• receiving at least one visual behavior parameter indicative of the real time visual behavior of the user, and
• determining the migraine risk of the user based on the analysis of the at least one visual behavior parameter,
wherein the computer executable instructions further comprise instructions for:
- providing at least one migraine history parameter indicative of the migraine history of the user, and
- updating the migraine risk based on at least one migraine history parameter, the migraine history parameter comprising data representative of the past level of pain of the user.

2. The component according to claim 1, wherein the visual behavior parameter relates at least to the eyelids activity of the user.

3. The component according to claim 1 or 2, wherein the computer executable instructions further comprise instructions for:
- receiving real-time physiological data relating to at least one real time parameter of the physiology of the user, and
- determining the migraine risk considering the real-time physiological data.

4. The component according to claim 3, wherein the real-time parameter of the physiology of the user relates to one or more of: sweating of the user, the pulse of the user, the temperature of the user, the breathing rhythm of the user, and muscle spasm of the user.

5. The component according to any of the preceding claims, wherein the computer executable instructions further comprise instructions for:
- receiving real-time environment data relating to at least one real-time parameter of the environment of the user, and
- determining the migraine risk considering the real-time environment data.

6. The component according to claim 5, wherein the real-time parameter of the environment of the user relates to the features of the light received by the user, said features comprising at least one of the temporal features, the spatial features, the spectral features, and the intensity of the light.

7. The component according to claim 5 or 6, wherein the real-time parameter of the environment of the user relates to one or more of: temperature, the noise of the environment of the user, and the time of the day.

8. The component according to any of the preceding claims, wherein the computer executable instructions further comprise instructions for:
- receiving life habits data and/or health profile data relating to at least one parameter of the life habits of the user and/or to at least one parameter of the health profile of the user, and
- adjusting the migraine risk based on the life habits data and/or on the health profile data.

9. The component according to claim 8, wherein the parameter of the life habits of the user relates to one or more of: the food habits of the user, the physical activity habits of the user, the sleeping habits of the user, and the rhythm of life of the user, and wherein the parameter of the health profile of the user relates to one or more of: the professional situation of the user, the personal situation of the user, the general state of health of the user, the gender of the user, the age of the user, and the ongoing migraine treatment of the user.

10. The component according to claim 9, wherein the parameter of migraine history is determined with measurements and/or with answers of the user to questionnaires.

11. A real-time visual behavior measuring device (20) comprising the user migraine
analysis component 10, according to any of the preceding claims, being embedded into the device and
at least one sensor configured to measure in real time at least one visual behavior parameter indicative of the visual behavior of the user and a communication unit (38) configured to communicate the measured real time visual behavior parameter to a user migraine analysis component.

12. The real-time visual behavior measuring device according to claim 11, wherein the real-time visual behavior measuring device (20) is a head mounted device arranged to be mounted on the head of the user.

## Patentansprüche

1. Anwenderspezifische Migräneanalysekomponente (10), umfassend:
- einen Speicher (14), der konfiguriert ist, computerausführbare Anweisungen zu speichern; und
- einen Prozessor (16) zum Ausführen der computerausführbaren Anweisungen, wobei die computerausführbaren Anweisungen Anweisungen umfassen zum:
• Empfangen mindestens eines visuellen Verhaltensparameters, der das visuelle Verhalten des Anwenders in Echtzeit anzeigt, und
• Bestimmen des Migränerisikos des Anwenders basierend auf der Analyse mindestens eines visuellen Verhaltensparameters,
wobei die computerausführbaren Anweisungen ferner Anweisungen umfassen zum:
- Bereitstellen mindestens eines Migräneanamneseparameters, der auf die Migräneanamnese des Anwenders hinweist, und
- Aktualisieren des Migränerisikos basierend auf mindestens einem Migräneanamneseparameter, wobei der Migräneanamneseparameter Daten umfasst, die für das frühere Schmerzniveau des Anwenders repräsentativ sind.

2. Komponente nach Anspruch 1, wobei sich der visuelle Verhaltensparameter zumindest auf die Lidaktivität des Anwenders bezieht.

3. Komponente nach Anspruch 1 oder 2, wobei die computerausführbaren Anweisungen ferner Anweisungen umfassen zum:
- Empfangen physiologischer Echtzeitdaten, die sich auf mindestens einen Echtzeitparameter der Physiologie des Anwenders beziehen, und
- Bestimmen des Migränerisikos unter Berücksichtigung der physiologischen Echtzeitdaten.

4. Komponente nach Anspruch 3, wobei sich der Echtzeitparameter der Physiologie des Anwenders auf eines oder mehrere der folgenden bezieht: Schwitzen des Anwenders, den Puls des Anwenders, die Temperatur des Anwenders, den Atemrhythmus des Anwenders und Muskelkrämpfe des Anwenders.

5. Komponente nach einem der vorhergehenden Ansprüche, wobei die computerausführbaren Anweisungen ferner Anweisungen umfassen zum:
- Empfangen von Echtzeitumgebungsdaten, die sich auf mindestens einen Echtzeitparameter der Umgebung des Anwenders beziehen, und
- Bestimmen des Migränerisikos unter Berücksichtigung der Echtzeitumgebungsdaten.

6. Komponente nach Anspruch 5, wobei sich der Echtzeitparameter der Umgebung des Anwenders auf die Merkmale des vom Anwender wahrgenommenen Lichts bezieht, wobei die Merkmale mindestens eines von den zeitlichen Merkmalen, den räumlichen Merkmalen, den spektralen Merkmalen und der Intensität des Lichts umfassen.

7. Komponente nach Anspruch 5 oder 6, wobei sich der Echtzeitparameter der Umgebung des Anwenders auf eines oder mehrere der folgenden bezieht: Temperatur, das Rauschen der Umgebung des Anwenders und die Tageszeit.

8. Komponente nach einem der vorhergehenden Ansprüche, wobei die computerausführbaren Anweisungen ferner Anweisungen umfassen zum:
- Empfangen von Daten zu den Lebensgewohnheiten und/oder Daten zum Gesundheitsprofil, die sich auf mindestens einen Parameter der Lebensgewohnheiten des Anwenders und/oder auf mindestens einen Parameter des Gesundheitsprofils des Anwenders beziehen, und
- Anpassen des Migränerisikos basierend auf den Daten zu den Lebensgewohnheiten und/oder den Daten zum Gesundheitsprofil.

9. Komponente nach Anspruch 8, wobei sich der Parameter der Lebensgewohnheiten des Anwenders auf eines oder mehrere der folgenden bezieht: die Ernährungsgewohnheiten des Anwenders, die körperlichen Aktivitätsgewohnheiten des Anwenders, die Schlafgewohnheiten des Anwenders und den Lebensrhythmus des Anwenders, und wobei sich der Parameter des Gesundheitsprofils des Anwenders auf eines oder mehrere der folgenden bezieht: die berufliche Situation des Anwenders, die persönliche Situation des Anwenders, den allgemeinen Gesundheitszustand des Anwenders, das Geschlecht des Anwenders, das Alter des Anwenders und die laufende Migränebehandlung des Anwenders.

10. Komponente nach Anspruch 9, wobei der Parameter der Migräneanamnese mittels Messungen und/oder mittels Antworten des Anwenders auf Fragebögen bestimmt wird.

11. Echtzeitmessvorrichtung für das visuelle Verhalten (20), umfassend die anwenderspezifische Migräneanalysekomponente 10 nach einem der vorhergehenden Ansprüche, die in die Vorrichtung eingebettet ist und mindestens einen Sensor, der konfiguriert ist, in Echtzeit mindestens einen visuellen Verhaltensparameter zu messen, der auf das visuelle Verhalten des Anwenders hinweist, und eine Kommunikationseinheit (38), die konfiguriert ist, den gemessenen visuellen Echtzeitverhaltensparameter an eine anwenderspezifische Migräneanalysekomponente zu übermitteln.

12. Echtzeitmessvorrichtung für das visuelle Verhalten nach Anspruch 11, wobei die Echtzeitmessvorrichtung für das visuelle Verhalten (20) eine kopfmontierte Vorrichtung ist, die am Kopf des Anwenders montiert werden kann.

## Revendications

1. Composant d'analyse de migraine d'utilisateur (10), comprenant :
- une mémoire (14) configurée pour stocker des instructions exécutables par ordinateur ; et
- un processeur (16) pour exécuter les instructions exécutables par ordinateur, les instructions exécutables par ordinateur comprenant des instructions pour :
• recevoir au moins un paramètre de comportement visuel indicatif du comportement visuel en temps réel de l'utilisateur, et
• déterminer le risque de migraine de l'utilisateur sur la base de l'analyse de l'au moins un paramètre de comportement visuel,
les instructions exécutables par ordinateur comprenant en outre des instructions pour :
- fournir au moins un paramètre d'historique de migraine indicatif de l'historique de migraine de l'utilisateur, et
- mettre à jour le risque de migraine sur la base au moins d'un paramètre d'historique de migraine, le paramètre d'historique de migraine comprenant des données représentatives du niveau antérieur de douleur de l'utilisateur.

2. Composant selon la revendication 1, le paramètre de comportement visuel concernant au moins l'activité des paupières de l'utilisateur.

3. Composant selon la revendication 1 ou 2, les instructions exécutables par ordinateur comprenant en outre des instructions pour :
- recevoir des données physiologiques en temps réel relatives à au moins un paramètre en temps réel de la physiologie de l'utilisateur, et
- déterminer le risque de migraine en tenant compte des données physiologiques en temps réel.

4. Composant selon la revendication 3, le paramètre en temps réel de la physiologie de l'utilisateur concernant un ou plusieurs des paramètres suivants : transpiration de l'utilisateur, pouls de l'utilisateur, température de l'utilisateur, rythme respiratoire de l'utilisateur, et spasme musculaire de l'utilisateur.

5. Composant selon l'une quelconque des revendications précédentes, les instructions exécutables par ordinateur comprenant en outre des instructions pour :
- recevoir des données environnementales en temps réel relatives à au moins un paramètre en temps réel de l'environnement de l'utilisateur, et
- déterminer le risque de migraine en tenant compte des données environnementales en temps réel.

6. Composant selon la revendication 5, le paramètre en temps réel de l'environnement de l'utilisateur concernant les caractéristiques de la lumière reçue par l'utilisateur, lesdites caractéristiques comprenant au moins des caractéristiques parmi les caractéristiques temporelles, les caractéristiques spatiales, les caractéristiques spectrales et l'intensité de la lumière.

7. Composant selon la revendication 5 ou 6, le paramètre en temps réel de l'environnement de l'utilisateur concernant un ou plusieurs des paramètres suivants : température, bruit de l'environnement de l'utilisateur, et heure du jour.

8. Composant selon l'une quelconque des revendications précédentes, les instructions exécutables par ordinateur comprenant en outre des instructions pour :
- recevoir des données sur les habitudes de vie et/ou des données de profil de santé relatives à au moins un paramètre des habitudes de vie de l'utilisateur et/ou à au moins un paramètre du profil de santé de l'utilisateur, et
- ajuster le risque de migraine en fonction des données sur les habitudes de vie et/ou sur les données de profil de santé.

9. Composant selon la revendication 8, le paramètre des habitudes de vie de l'utilisateur concernant un ou plusieurs des éléments suivants : les habitudes alimentaires de l'utilisateur, les habitudes d'activité physique de l'utilisateur, les habitudes de sommeil de l'utilisateur et le rythme de vie de l'utilisateur, et le paramètre du profil de santé de l'utilisateur concernant un ou plusieurs des éléments suivants : la situation professionnelle de l'utilisateur, la situation personnelle de l'utilisateur, l'état de santé général de l'utilisateur, le sexe de l'utilisateur, l'âge de l'utilisateur, et le traitement de migraine en cours de l'utilisateur.

10. Composant selon la revendication 9, le paramètre d'historique de migraine étant déterminé par des mesures et/ou par des réponses de l'utilisateur à des questionnaires.

11. Dispositif de mesure de comportement visuel en temps réel (20) comprenant le composant d'analyse de migraine d'utilisateur (10), selon l'une quelconque des revendications précédentes, incorporé dans le dispositif et au moins un capteur configuré pour mesurer en temps réel au moins un paramètre de comportement visuel indicatif du comportement visuel de l'utilisateur et une unité de communication (38) configurée pour communiquer le paramètre de comportement visuel en temps réel mesuré à un composant d'analyse de migraine d'utilisateur.

12. Dispositif de mesure de comportement visuel en temps réel selon la revendication 11, le dispositif de mesure de comportement visuel en temps réel (20) étant un dispositif monté sur la tête agencé pour être monté sur la tête de l'utilisateur.
